# EUROPEAN PATENT APPLICATION

(11) **EP 2 108 953 A1**
(43) Date of publication of application: **14.10.2009**
(21) Application number: 08251379.7
(22) Date of filing: 09.04.2008
(51) Int. Cl.: G01N 33/20

(54) **A tool for determining metal quality**

(71) Applicant: FOSECO INTERNATIONAL LIMITED, Tamworth, Staffordshire B78 3TL (GB)
(72) Inventor: The designation of the inventor has not yet been filed
(74) Representative: Debled, Thierry

(57) **Abstract**

A portable measuring tool for determining the quality of molten metal, the tool having a body comprising a filling portion for submersion in the molten metal in use, the filling portion comprising a filter to allow ingress of molten metal into the filling portion after at least partial submersion of the filling portion in molten metal, the tool further comprising a measuring device to measure molten metal characteristics in the filling portion, wherein the body is buoyant in the molten metal and a datum is provided on the tool for use by the measuring device in determining a molten metal characteristic in the filling portion, thereby to enable an indication of metal quality to be determined.

## Description

The invention relates to a tool for determining metal quality and a method for determining metal quality when the metal is molten, for example before casting.

For many applications, it is important to detect and measure inclusions above 50 µm as these will have deleterious effects on the final product. At present, there are a number of ways of detecting such particles, but most of these require removal of a molten metal sample for analysis. Methods such as Prefil and PoDFA involve removing a molten metal sample and passing it under pressure (Prefil) or using a vacuum (PoDFA) through a fine filter. In these techniques, inclusions are caused to build up the filter forming a cake. Sufficient metal is passed through the filter during the test such that the local increase in concentration of inclusions is typically 5000 to 10000 times. At the end of the test some liquid metal is allowed to remain above the filter, encapsulating the inclusions and allowed to solidify. This 'residue' can be cut up, metallographically examined in the laboratory and analyzed using standard techniques to identify and quantify the different inclusions present. The procedure requires a skilled (trained) metallographer and takes a minimum of 2 hours for each analysis. More typically it may be days or weeks before the results are returned to the originator and therefore the technique is not suitable for in-line process control.

One variant of the pressure filtration technique (Prefil) provides an output of the filtration rate curve during a 150 second test. This output is obtained in real time and the shape of the curve is compared with a database of curves to give the operator an immediate semi quantitative idea of the metal quality of the liquid metal, and can therefore be used to provide a 'go: no-go' measure of metal quality. However, a metal sample must still be removed from the line, thus causing time delays.

A further disadvantage of the Prefil and PoDFA techniques is that, in filtering the molten metal, cake formation occurs in the filter. This therefore renders the filter unsuitable for use in further measurements.

A further known technique for the measurement of molten metal quality is the reduced pressure test, (RPT), or density index measurement. This technique involves taking two small (∼100gm) liquid metal samples with a pre-heated ladle, transferring the metal to a pre-heated metal cup and then allowing one sample to solidify normally in air at ambient pressure and solidifying the second sample under a reduced pressure by sealing in a (vacuum) chamber. It is well know that gas (for example hydrogen) dissolved in liquid metal nucleates preferentially on oxide films and certain other inclusions in molten metals when solidified under reduced pressure. This results in large gas bubbles being evolved and trapped in the sample during solidification with a commensurate decrease in sample density. A measurement of the density ratio of the two samples can be used as an indirect indicator of the combined gas and inclusion content of the metal. However, RPT is highly sensitive to the initial gas content as well as the sampling technique and can be operator dependent and therefore does not always give an unambiguous or reliable measure of inclusion content. Furthermore, the RPT is insensitive to the presence of certain other types of inclusion such as for example, intermetallic or grain refiner particles that may be present or may precipitate from the melt. Such particles can grow and /or agglomerate to sizes in excess of 50microns and may be deleterious to the quality of some castings or other products. In addition, a measurement takes 5-10 mins, and so is time consuming.

A further known technique for the detection of inclusions in molten metal is called Limca. Limca works by dipping a probe (glass tube) into the molten metal. Vacuum is used to draw metal into the probe through a single 300 µm hole in the side of the probe. Whilst the metal is flowing through the hole, a large electric current is passed between two electrodes, one either side of the hole. This causes an electric field to be set up that is highly concentrated as it passes through the hole. The electrical potential between the electrodes is continuously monitored and whenever a particle with an electrical conductivity different to that of the molten metal flows through the hole, the device detects a perturbation in the electric field. The size of the perturbation is proportional to the inclusion size passing through the hole. In this way a profile of inclusion size and number can be established. However, this technique is limited to inclusions in the range of 10-300 microns.

In a further known technique, an indication of melt cleanliness can be determined directly from metal in the melt by plunging a tube containing a filter in its base below the molten metal surface and monitoring the change in filtrate volume in the tube over time. A less clean melt contains a higher inclusion concentration and so greater cake formation occurs in the filter resulting in a decreased filtration rate compared to a cleaner melt. The tube is sealed and attached to a vacuum pump to ensure that the molten metal flows through the filter under constant filtration pressure. The displacement of the tube due to the change in buoyancy on filling with molten metal is measured using a balance with a fulcrum outside the metal bath. Therefore, although metal taken directly from the melt is used, some equipment is located outside the melt, making the equipment bulky with several different parts to control, and not easily portable. In addition, the requirement of constant pressure in the tube involves using complex and expensive equipment.

According to the present invention there is provided a portable measuring tool for determining the quality of molten metal, the tool having a body comprising a filling portion for submersion in the molten metal in use, the filling portion comprising a filter to allow ingress of molten metal into the filling portion after at least partial submersion of the filling portion in molten metal, the tool further comprising a measuring device to measure molten metal characteristics in the filling portion, wherein the body is buoyant in the molten metal and a datum is provided on the body for use by the measuring device in determining a molten metal characteristic (such as the amount) in the filling portion, thereby to enable an indication of metal quality to be determined.

As the datum is provided on the body, it is self-locating in relation to the level of the molten metal, and therefore its height does not need to be readjusted if the height of the molten metal in the metal bath changes. The equipment is therefore convenient as it is simple and portable, and so can quickly and easily be transported to different locations in a plant and be ready to use with minimal set-up time.

Preferably the body comprises floatation means to enable buoyancy and a consistent plunge depth of the filter in the molten metal.

Positioning the filter at the correct depth is therefore quick and simple.

Preferably the filling portion is open to the atmosphere in use so that the pressure inside is the ambient pressure above the molten metal such as atmospheric pressure, thus enabling molten metal to enter the filling portion as a result of the difference between the head (i.e. height) of molten metal around the tool and the head (or height) of metal in the filling portion, in other words the metallostatic head.

Therefore, no extra equipment is needed to keep the pressure in the filling portion constant. This again improves the portability of the tool. Further, it allows measurements to be taken in a short time, as minimal preparation time is needed before taking a measurement.

According to a second aspect of the present invention there is provided a tool for determining the quality of molten metal, the apparatus comprising a body for at least partial submersion in the molten metal, the body comprising a filling portion, a filter being provided to enable ingress of molten metal into the filling portion on at least partial submersion of the body, the filling portion is open so that the pressure inside is the ambient pressure above the molten metal such as atmospheric pressure, thus enabling molten metal to enter the filling portion as a result of the difference between the head of molten metal around the tool and the metal in the filling portion, in other words the metallostatic head, a measuring device being provided to measure the molten metal characteristics in the filling portion, thereby to enable an indication of metal quality to be determined.

Preferably the measuring device comprises means to measure the amount of molten metal in the filling portion. Preferably the tool enables the time to at least partially fill the filling portion to be determined.

Preferably a datum is provided on the body for use by the measuring device in determining the amount of metal in the filling portion. Thus, the equipment is simple, compact, and easily portable.

Preferably the tool includes location means to locate the body at a specific position relative to the level of the molten metal to be measured at the start of each measurement. Preferably the location means is a floatation device formed as part of the body. The tool is therefore automatically kept at the correct position relative to the surface of the molten metal if the surface of the molten metal alters during the measurement or between measurements.

The filling portion may be able to move relative to the body. Preferably the filling portion is attached to the body via a hinge and preferably the hinge allows substantially vertical movement of the filling portion relative to the body. Therefore, as the filling portion fills, it is able to move down into the metal bath, as a result of its reduced buoyancy.

Preferably the measuring device is adapted to measure displacement of the filling portion relative to the body.

Preferably the means to measure displacement is provided substantially opposite the hinge point. This allows the maximum possible displacement to be measured, thus increasing accuracy.

The means to measure displacement may comprise a force sensor provided on the body and a movement transfer member substantially vertically upstanding from the filling portion, the member being in contact with the force sensor. Therefore, as the filling portion fills with molten metal, and moves downwards into the molten metal bath due to reduced buoyancy, the force applied to the force sensor by the movement transfer member will decrease, thus providing a means to measure the filling of the filling portion.

The filling portion may comprise two parts: a hinged member attached to the hinge, and a removable hollow portion, the filter being provided on the hollow portion.

Preferably, the hinged member defines an aperture for location of the hollow portion. Preferably, the movement transfer member is provided on the hinged member.

Therefore, the hollow portion, into which molten metal flows, can be easily removed and replaced, if necessary.

Preferably the tool comprises a transducer for converting the relative displacement of the filling portion relative to the body as a result of the change of buoyancy of the filling portion on filling to an output signal representative of the amount of molten metal in the filling portion.

The filling portion may be fixed relative to the body.

The datum may be provided as a first reference against subsequent filling by the molten metal, and subsequent references can be used to determine the amount of molten metal in the filling portion in a non-continuous measuring process.

The datum may comprise a pair of electrical contacts. The electrical contacts may be located at the same height above the filter. The electrical contacts may be located at different heights above the filter. The contacts therefore can show when the molten metal has reached a certain height within the filling portion. More than one pair of electrical contacts may be provided. Combined with timing the filling process, the output of the electrical contacts can be used as the measuring device to determine the amount of molten metal in the filling portion at different points in time.

The measuring device may comprise a resistance element placed substantially vertically in the filling portion. The resistance element may comprise a tube, with a wire being passed down the inside of the tube and emerging from the bottom, then being coiled around the outside of the tube to the top of the tube, the tube being sealed at the bottom to prevent entry of molten metal into the tube, both ends of the wire being connected to a circuit. As the level of the molten metal in the filling portion increases in height a direct conduction path is provided, bypassing the high resistance coil. Therefore, the resistance of the circuit gives an indication of the height of the molten metal in the filling portion.

A float may be provided inside the filling portion, the float being suitable for floatation on top of the molten metal which fills the filling portion, and preferably a rod is attached to the float extending substantially vertically upwards from the float.

The measuring means may comprise an optical sensor, the optical sensor being able to detect displacement of the float and/or rod when present relative to the sensor.

The location means may be provided by at least one electrical contact on the outside of the body. Therefore, the tool of the second aspect can be located at the desired submersion depth without a float.

Preferably the filling portion can be filled with at least 1 kg of molten metal, more preferably at least 1.5kg of molten metal, and most preferably at least 2 kg of molten metal. The more metal that can be filtered during the test, the more meaningful the results obtained.

The filter may be masked to define the area of filter through which molten metal can flow.

The unmasked filter area on the outside of the filling portion is preferably equivalent to a circular area with a diameter of between about 10 mm and about 30 mm.

Preferably the filter pore size, percentage porosity of the filter, area of the mask and depth of submersion of the filter are such that the filling portion can fill sufficiently for metal quality to be determined in less than 60 s, preferably less than 30 s.

The filter may have a pore size or porosity equivalent to a pore size of between about 300 µm and about 1500 µm.

The filter may be a ceramic foam filter, preferably with a pores per linear inch value of between 35 and 55, (equivalent to a pore size of between about 300 µm and about 500 µm). The surface of the filter inside the filling portion available for molten metal to pass through may be smaller than the area defined on the outside of the filling portion.

The filter may be a pressed or extruded filter, preferably with a pore size of between about 800 µm and about 1250 µm.

The filter may be removable, and preferably is provided on a removable element. The removable element may define the area of the filter through which molten metal can flow.

Preferably the filter is positioned so as to enable egress of liquid from the filling portion, thus backflushing the filter. Preferably, the filling portion is adapted such that backflushing of the filter occurs when the tool is removed from the molten metal. Preferably the tool is adapted such that substantially uniform backflushing of the filter occurs automatically during use, and preferably the filter is positioned such that substantially uniform backflushing occurs irrespective of the rate of removal of the tool from the molten metal bath.

Substantially uniform backflushing of the filter ensures the whole of the filter surface is adequately cleaned of any build up of particles. Filling times will also remain consistent over subsequent filling cycles for molten metal of consistent quality. As the filter is backflushed automatically substantially uniformly across the filter, irrespective of the rate of removal of the tool from the molten metal, the removal rate does not need to be controlled. Therefore, cake formation in the filter can be minimised, and a consistent filling time over repeated filling and transfer cycles ensured, without having to reduce the rate of removal of the tool.

Preferably the tool is adapted such that egress of the molten metal from the tool is quiescent, and preferably such that egress of molten metal from the tool occurs substantially with the filter submerged in the molten metal.

Quiescent egress from the tool helps to minimise the entrainment of air, and hence the formation of oxide films and bifilms, which would decrease the quality of the unfiltered metal.

Preferably the filter is positioned so that substantially the whole of the filter surface is backflushed for substantially the whole of the backflushing period. This therefore achieves efficient backflushing and ensures that there is minimal build up of particles over the whole surface of the filter.

Preferably, the tool is adapted such that, in use, the egress of metal is substantially uniform across the filter. Preferably the tool is adapted such that, in use, the filter is of substantially uniform thickness in the direction of egress. The tool may be adapted such that the filter is substantially horizontal during backflushing. A substantially horizontal filter ensures that substantially the whole of the filter surface is backflushed for substantially the whole of the backflushing period. In addition, a substantially horizontal filter has the added benefit that gravity will act substantially in the direction of shortest distance through the filter.

The filter may be an integral part of the filling portion. When the filter is provided on a removable element, it may become detached on backflushing. If the filter is an integral part of the tube, it will not become detached on backflushing and so backflushing is likely to be more effective.

The tool may further comprise a placement arm for attachment at one end to the body, the placement arm allowing placement of the tool in the molten metal. The operator therefore does not have to get too close to the molten metal bath. Preferably means to indicate the quality of the metal is provided on the placement arm. Therefore, the tool is convenient as measurement and retrieval of results can occur in a single location.

A yet further aspect of the invention provides a tool for characterising molten metal, comprising a float, a chamber depending from the float and defining an inlet at the lower end, the inlet being covered by a filter, the tool further comprising a measuring device having a sensor and a datum.

Another aspect of the invention provides a tool for characterising molten metal comprising a body for insertion into molten metal, a displaceable element movable in accordance with amount of molten metal in the body, and a measurement device comprising a wireless sensor for determining the displacement of the movable element thereby enabling characterisation of the molten metal.

Preferably the measurement device comprises an optical sensor adapted to determine movement of the displaceable element which in one embodiment can comprise an optically detectable reference object such as a rod having a reflective surface. The displaceable element further can comprise means to enable displacement due to the molten metal in the body, such as a float adapted to remain buoyant in molten metal and hence move the reference object due to an increase in molten metal in the body.

According to a further aspect of the present invention there is provided a method for determining the quality of molten metal using an apparatus comprising a body and a filling portion, a filter being provided to enable ingress of molten metal into the filling portion, the method comprising the steps of:
at least partially submerging the body in the molten metal to a set plunge depth;
allowing the filling portion to at least partially fill with molten metal between a first reference and at least one additional reference by means of the metallostatic pressure caused by the at least partial submersion of the body;
measuring the time to fill the filling portion; using the time to fill the filling portion to provide an indication of the metal quality.

The metal quality, may be determined at a location remote from the body, and transferred to the body for display to the user.

The metal quality may be determined in less than 60 s, or even and preferably less than 30 s.

Seven embodiments of the tool of the invention will now be described by way of example and with reference to the accompanying drawings, in which:
Fig. 1 is a side view of the tool of embodiment 1, with the placement arm attached, resting on a stand;
Fig. 2 is a side view of the tool of Fig. 1 with the foot attached, but without the placement arm;
Fig. 3 is a perspective view of the tool of Fig. 1 without the foot attached, and without the placement arm;
Fig. 4 is a view from above of the float of the tool of Fig. 1, with some of the sheeting removed from the top;
Fig. 5 is a cross section view of the tool of figure 1, without the placement arm attached;
Fig. 6 is a perspective view from above of the tool of Fig. 1, showing the hinge plate, world plate, shoe, and foot;
Fig. 7 is a perspective view from below of the tool of Fig. 1, showing the hinge plate, world plate, shoe, and foot;
Fig. 8 is a cross section view of the tool of Fig. 1, showing the hinge plate, world plate, shoe, and foot;
Fig. 9 is a cross section view as shown in Fig. 8, with the filling portion included;
Fig. 10 illustrates the filter arrangement of the filling portion of the tool of Fig. 1;
Fig. 11 is a perspective view of the hinge arrangement of the tool of Fig. 1;
Fig. 12 a to d is a schematic representation of the tool of Fig. 1 illustrating the change in buoyancy force on the load cell when the tool of Fig. 1 is placed in molten metal;
Fig. 13 illustrates the electronics system of the tool of Fig. 1;
Fig. 14 is a graph showing the load output measured by the tool of Fig. 1 when placed in molten aluminium;
Fig. 15 illustrates the tool of the second embodiment;
Fig. 16 illustrates the tool of the second embodiment mounted on a placement frame, and ready to lower into molten metal;
Fig. 17 illustrates the tool of the second embodiment mounted on a placement frame, and lowered into molten metal;
Fig. 18 illustrates in cross section the tool of the third embodiment;
Fig. 19a to 19e illustrates filling and emptying of the tool of Fig. 14;
Fig. 20 illustrates the tool of the fourth embodiment in cross section;
Fig. 21 is a perspective view of the tool of Fig. 16;
Fig. 22 is a cross sectional representation of the tool of the fifth embodiment;

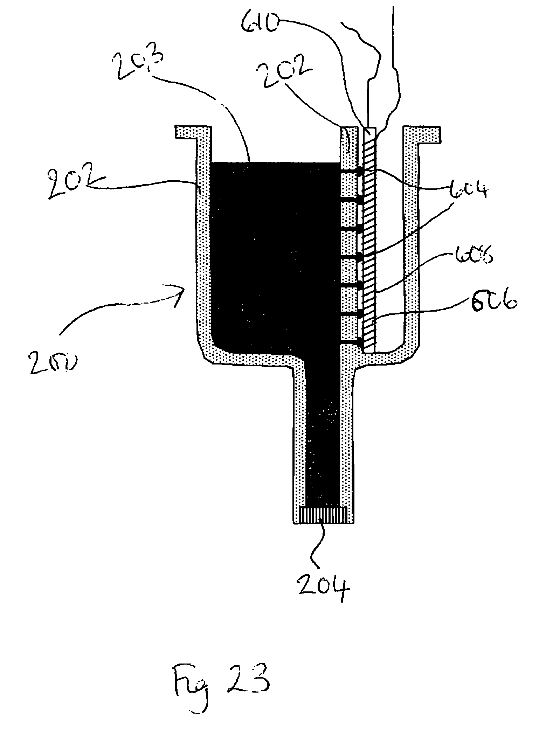

Fig. 23 is a cross sectional representation of the tool of the sixth embodiment; and
Fig. 24a and 24b illustrates the tools of the seventh embodiment before molten metal has entered the filling portion and with molten metal in the filling portion.

### Embodiment 1

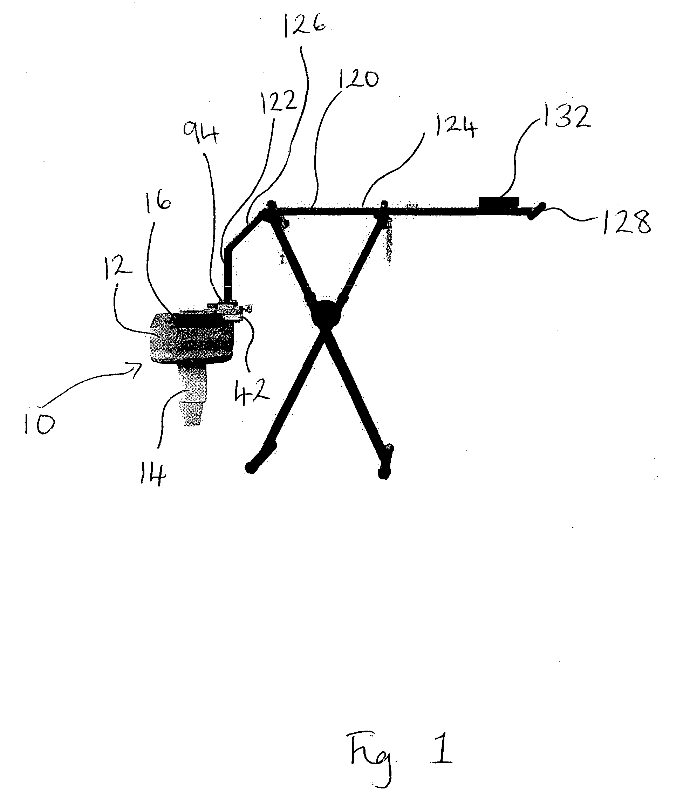

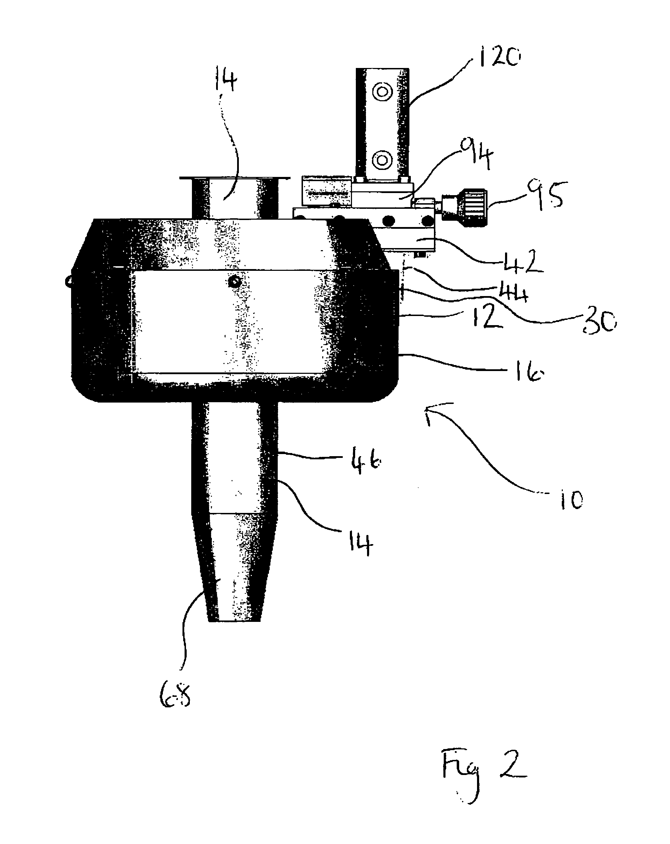

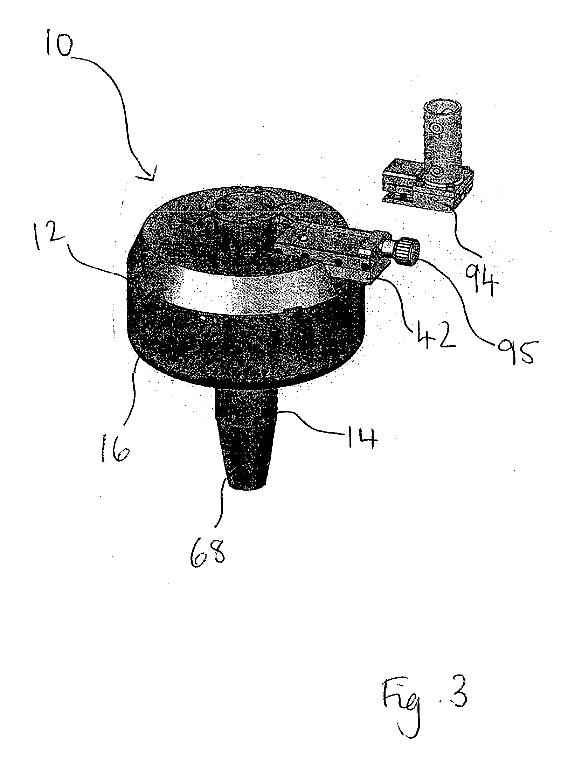

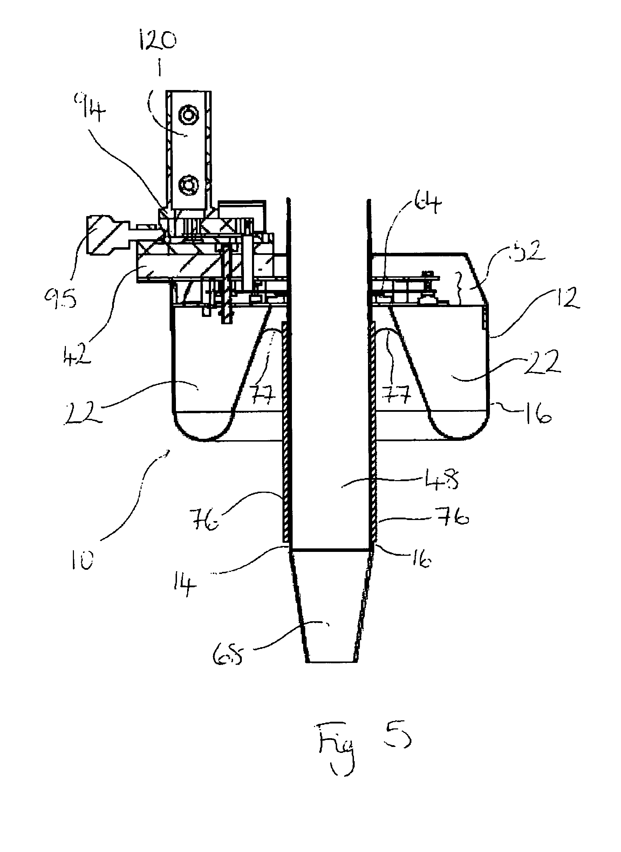

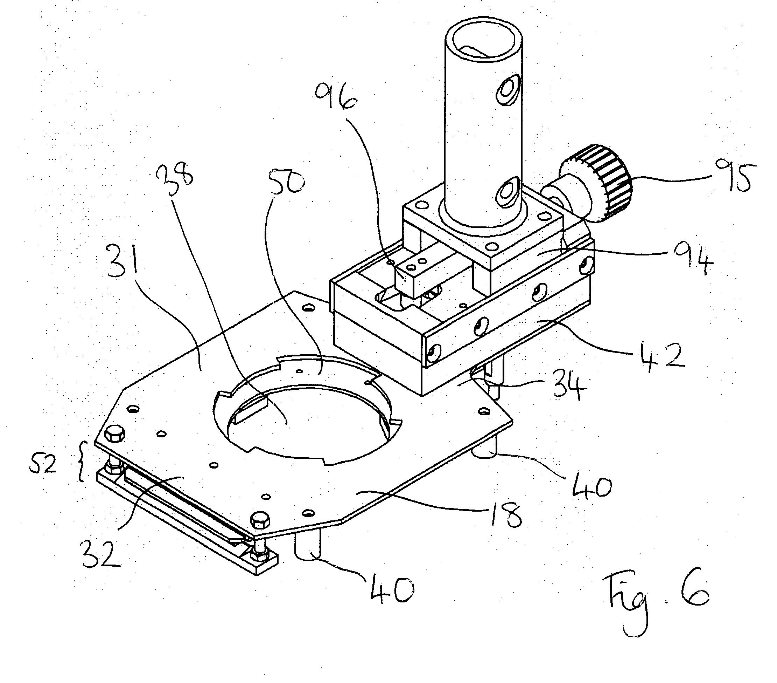

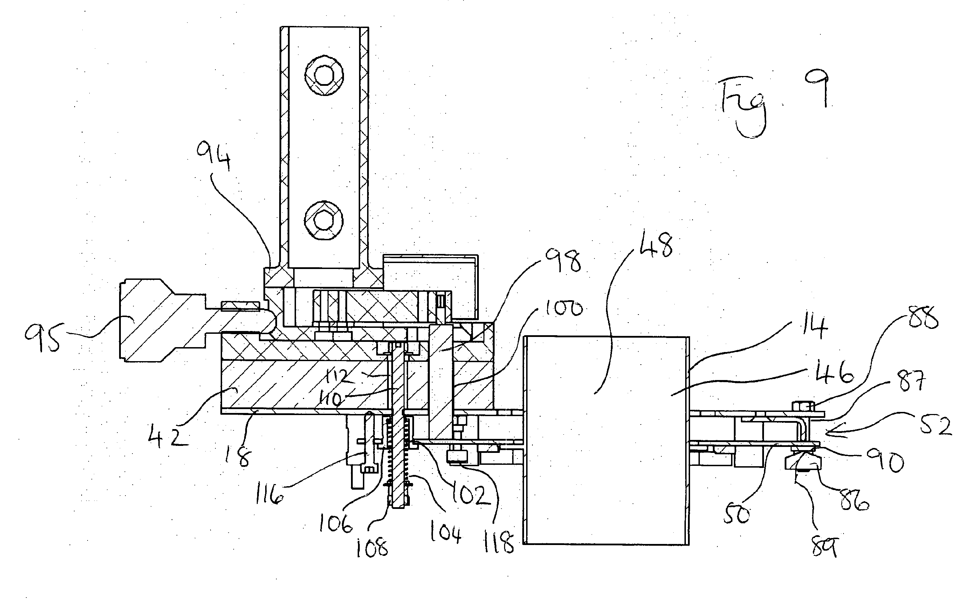

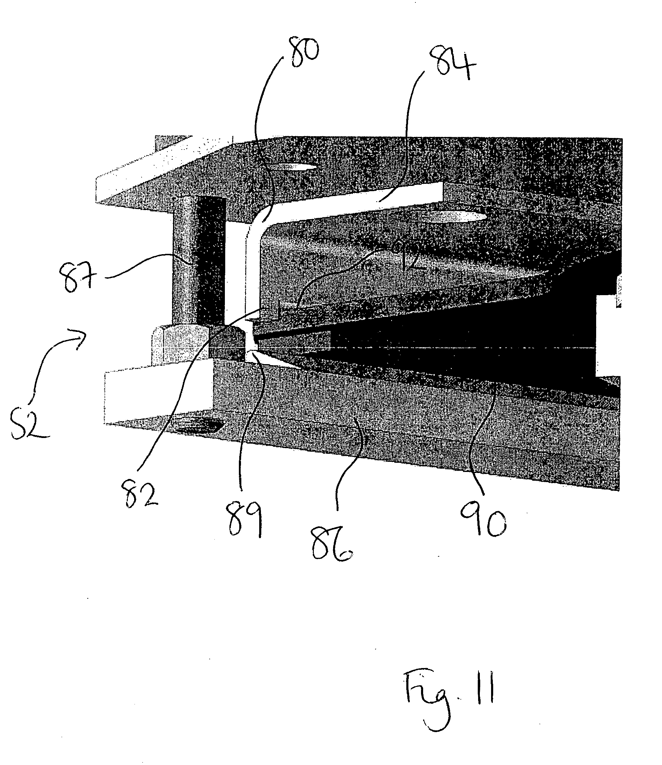

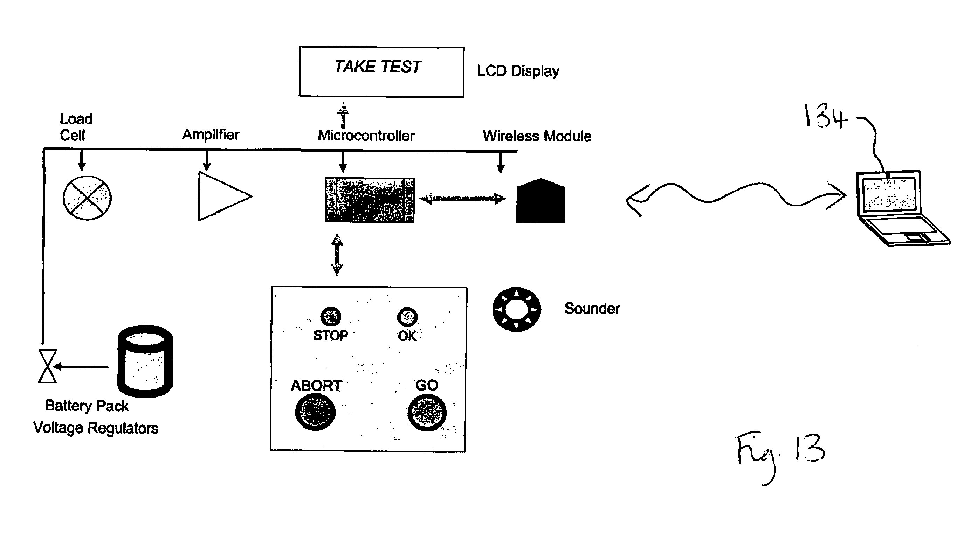

Figures 1 to 13 illustrate the tool or probe 10 of the first embodiment. The probe 10 comprises a body 12 and a filling portion 14.

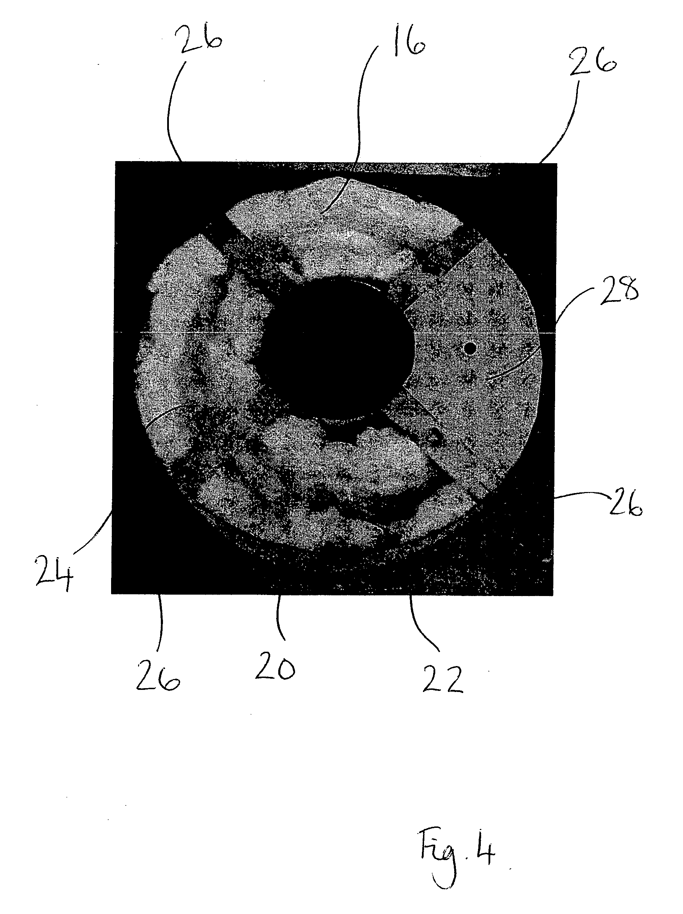

The body 12 is circular and comprises a float 16 and a reference plate 18. The float 16 is shown in Fig. 4. It is annular in shape, with a shell 20 formed from sheets of mild steel. The shell defines a cavity 22 which is filled with insulating wool 24. Four equally spaced radial struts 26 are provided on the upper surface of the float 16 to help stiffen the structure. The gaps in between the struts are covered with sheets 28 of mild steel, which overlap the outer edge 30 of the float 16 to provide further stiffening. The reference plate 18 comprises a rectangular portion 31, which tapers at one end 32. At the other end 34 of the reference plate 18 there is a rectangular protrusion 36, about half the width of the reference plate 18, and located centrally at the end 34 of the reference plate 18. The reference plate defines an aperture 38. The reference plate 18 is made from sheet gauge plate steel, which has high stiffness and high strength, as it is important that flexing of the reference plate 18 is minimised. To support the reference plate 18, four tube spacers 40 are provided between the reference plate 18 and the float 16, near the corners of the rectangular portion 31 of the reference plate 18. A shoe 42 is located on top of the rectangular protrusion 36 of the reference plate 18. A bracket 44 is attached to the outer edge 30 of the float 16 to support the rectangular portion 36 of the reference plate 18.

The filling portion 14 comprises a hollow member which is a cylindrical tube 46 defining a bore 48, attached to a hinged member or hinge plate 50. The tube 46 is made from mild steel spray coated with boron nitride. The hinge plate 50 is fixed between the float 16 and the reference plate 18 by means of a hinge 52.

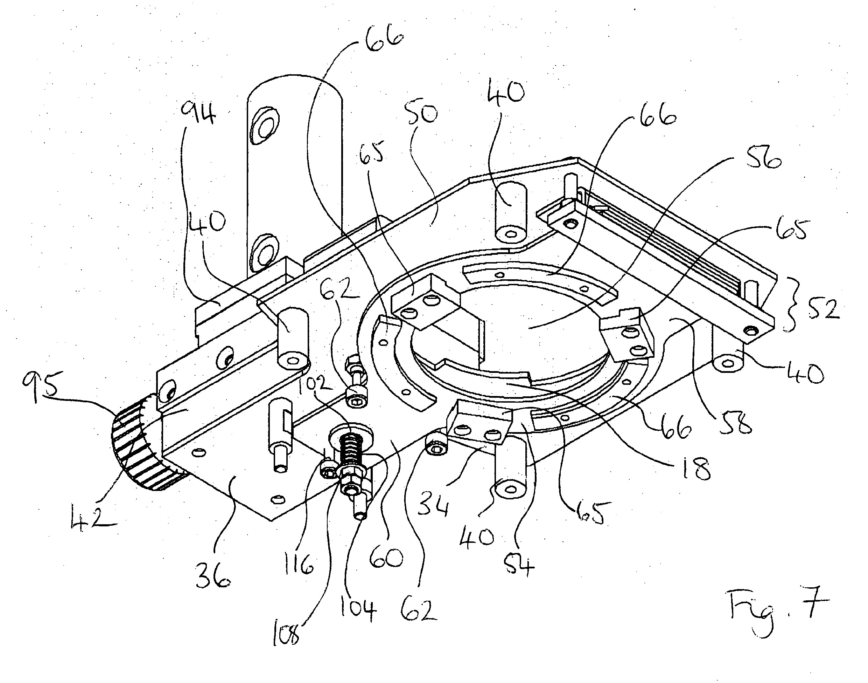

The hinge plate 50 is shown in Fig. 7. The hinge plate 50 is made from sheet gauge plate steel, as it is important to ensure flexing of the hinge plate is minimised. The hinge plate 50 has an annular portion 54, and thus defines an aperture 56, and two rectangular portions, forming a first protrusion 58 and a second protrusion 60, which protrude approximately equal distances from opposite sides of the outer edge of the annular portion 54. The first protrusion 58 is approximately twice as wide as the second protrusion 60. The first protrusion 58 is positioned underneath the end 32 of the reference plate 18 which is tapered. The hinge 52 is located across the first protrusion 58. The second protrusion 60 is positioned underneath the shoe 42. Stops 62 are provided either side of the second protrusion 60, close to the edge of the annular portion 54, to restrict sideways movement of the hinge plate 50.

The cylindrical tube 46 is arranged in the apertures 38, 56 of the reference plate 18 and the hinge plate 50, respectively. The tube 46 has three flanges 64 close to one end. The tube is inserted through the aperture from underneath, so that the flanges 64 contact the bottom of the hinge plate 50 in between three tube supports 65 which depend from the hinge plate 50. The tube is then twisted so that the flanges 64 lie between the tube supports 65 and the hinge plate 50. Correct location of the tube is ensured by three guiding members 66 in between the tube supports 65.

The tube 46 is open at the clamped end, and so the inside of the tube 46 is at ambient pressure. The tube 46 tapers at its free end 68. A cup 70, with a flat base 72 and tapered side 74, is fitted to the tapered end 68 of the tube 46, and held in place by a thin layer of molten metal 75 on the tapered end 68 applied by dipping the tapered end 68 of tube 46 in the molten metal bath. The cup 70 is, therefore, removable.

Part of the cylindrical portion of the tube 46 is surrounded by a floatation collar 76. The floatation collar 76 comprises a tube of low density ceramic material and is attached to the tube 46 using a ceramic paper gasket.

A filter 78 is provided in the base 72 of the cup 70. The filter 78 is a ceramic foam filter with 40 ppi (pores per linear inch) equivalent to a pore size of ∼410-450 µm, a diameter of 40 mm and a thickness of 15 mm. The cup 70 is formed by blowing powder of a lightweight insulating refractory material mixed with inorganic binder into a mould, in which the filter 78 is already in place. The cup 70 is oven or microwave cured to harden the binder. Producing the cup 70 in this way means that is dimensionally accurate with no leakage path around the filter 78. The cup 70 material is inert.

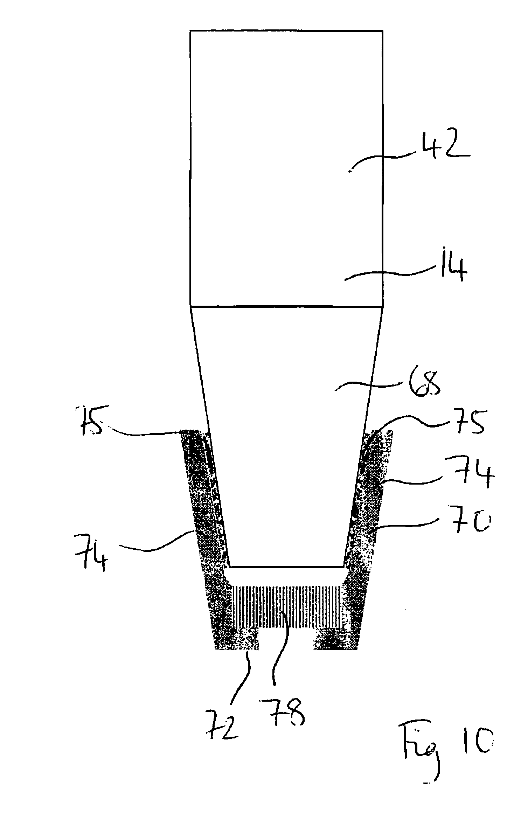

As shown in fig. 10, the base 72 of the cup 70 defines an aperture, the diameter of the aperture is 20 mm. When the cup 70 is correctly positioned in the tube 46, the filter 78 is parallel to the reference plate 18.

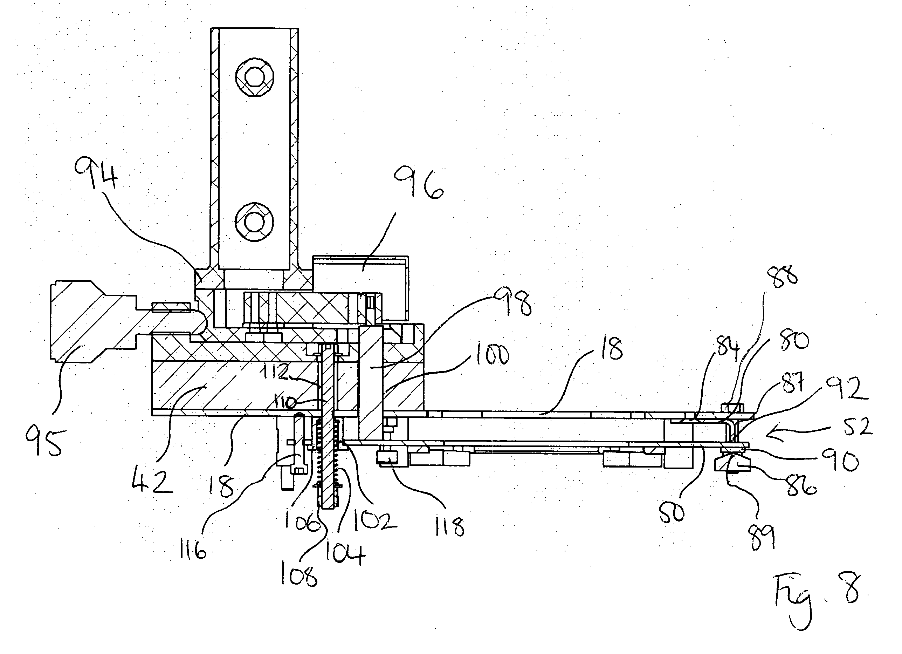

The hinge 52, fixing the hinge plate 50 between the float 16 and the reference plate 18, is shown in Fig. 8. 9, and 11. The hinge 52 is separated from the reference plate 18 by a bracket 80. The bracket is wider than the first protrusion 58, and stops 82 depending from the bracket 80 overlap the edges of the first protrusion 58 of the hinge plate 50, and keep the hinge plate 50 aligned with the reference plate 18. The bracket 80 is L-shaped in cross section, with one side 84 fixed to the bottom of the reference plate 18. The hinge plate 50 is held against the bracket 80 by means of a clamp bar 86, attached at either end to the reference plate by a bolt 87 and nut 88 arrangement. In cross section, the portion of the clamp bar 86 underneath the hinge plate 50 is raised to an apex 89, the apex 89 is opposite the edge of the bracket 80 which is in contact with the hinge plate 50. The hinge plate 50 is separated from the apex 89 of the clamp bar 86 by a strip of rubber 90. The edge 92 of the bracket 80 in contact with the hinge plate 50 is rounded. In use, the round edge 92 of the bracket 80 and the apex 89 of the clamp bar 86 allow the hinge plate 50 to be displaced vertically, by hinged movement, without flexing.

The shoe 42 receives a foot 94. The foot 94 is removable from the fixed shoe 42. The foot 94 is held in place in the shoe 42 by a clamping screw 95. The foot 94 includes a load cell 96. A movement transfer member 98 in the form of a pin is provided on the second protrusion 60 of the hinge plate, vertically upstanding from the hinge plate 50. The pin 98 is made from Macor^{™} ceramic. The shoe 42 and reference plate 18 define an aperture 100 beneath the load cell 96, through which the pin 98 passes, so that the pin 98 contacts the load cell 96.

An aperture 102 is provided in the second protrusion 60 of the hinge plate 50 and a spring 104 passes through the aperture 102. The spring 104 is retained by an upper retainer 106 attached to the hinge plate 50, and a lower retainer 108 attached to the sheet 28 of the float 16. A screw 110 is positioned inside the spring 104. The screw 110 contacts the lower retainer 108 and passes through an aperture 112 in the shoe 42 and the reference plate 18 above the aperture 102. The head of the screw 110 is accessible when the foot 94 is removed from the shoe 42, and the screw 110 can be turned to adjust the tension in the spring 104.

A further aperture 114 is provided in the hinge plate 50 between the spring 104 and the edge of the hinge plate 50. A screw 116 attached to the reference plate 18 and the sheet 28 of the float 16, passes through this aperture 114. The screw 116 helps to locate the hinge plate 50 horizontally and also limits vertical movement of the hinge plate 50. A further stop 118 is provided below the hinge plate 50 to limit downwards movement of the hinge plate 50.

The foot 94 is attached to one end of a placement member 120. The placement member 120 has a vertical section 122 extending from the foot 94, separated from a horizontal section 124 by a middle section 126 at approximately 45 degrees to both the vertical and horizontal sections 122, 124. A handle 128 is attached to the free end 130 of the horizontal section 124. A control box 132, with a screen, is attached to the horizontal section 124 in front of the handle 128. The control box 132 is connected to the load cell 96. The control box 132 connects wirelessly with a host PC 134, which is a remote location.

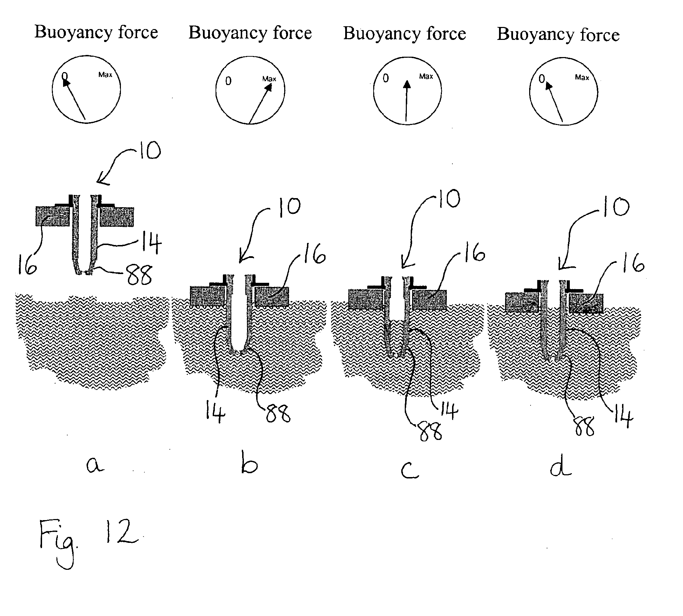

In use, the operator initialises the probe 10 by pressing a button on the control box 132 which activates a 3 second countdown, during which time the probe 10 is placed in the molten aluminium alloy bath. On initial placement there is an instantaneous upward force on the filling portion 14, as shown in Fig. 12b, due to the metallostatic head at the submersion depth. The world plate 18 is held fixed in position relative to the surface of the molten aluminium alloy bath by the float 16, and so the hinge plate 50 pivots upwards towards the reference plate 18 as a result of the buoyancy of the filling portion 14. The movement transfer pin 98 is therefore pushed towards the load cell 96, and the load cell 96 records an almost instantaneous positive load. The fact that the movement transfer pin 98 is made from Macor^{™} means that the pin 98 is dimensionally stable, and so the load is not affected by expansion of the pin 98 on heating. In addition, Macor^{™} exhibits low thermal conductivity, and so does not transmit heat to the load cell 96, which could damage the load cell 96.

The filling portion 14 fills with molten aluminium alloy, through the filter 78, and as it does so, the buoyancy of the filling portion 14 decreases. The anti-splash gasket 77 helps prevent molten aluminium alloy splashing onto the hinge plate. As the filling portion 14 is hinged, it is able to pivot downwards as the buoyancy decreases. A decreasing force is therefore measured by the load cell 96. It can be seen that the load cell 96, therefore, acts as a measuring device for detecting the amount of molten aluminium alloy in the filling portion 14. As molten aluminium alloy flows through the filter 78, cake formation will occur in the filter 78 as impurities greater than 50 µm are removed from the aluminium alloy and form particle bridges on the filter 78. Cake formation decreases the effective area of the filter 78 available for aluminium alloy to flow through to enter the filling portion 14, and so leads to increased time to fill the filling portion 14. The less clean the molten aluminium alloy passing through the filter 78, the greater the cake formation, and so the longer it will take to fill the filling portion 14. Time to fill the filling portion 14 can therefore be related to the cleanliness of the molten aluminium alloy sample.

The cleanliness of the aluminium alloy is determined by software on the host PC. The load measured by the load cell 96 is transferred wirelessly to the host PC 134 by the control box 114. On initial placement of the probe 10 in the molten aluminium alloy, if the probe 10 is functioning properly, the instantaneous load measured by the load cell 96 should be recognised by the software as exceeding a threshold load (Tᵢ). In response, the software initiates a timer. The start of filling time is deemed to be 0.1s before the timer is initiated.

As the tube 14 fills with molten aluminium alloy, it becomes less buoyant, and the tube 46 moves downwards, thus causing the hinge plate 50 to pivot downwards, and thus the load measured by the load cell 96 decreases. The software records the change in load with time.

The floatation collar 76 is engineered so that, when the filling portion is filled with 1.5 kg of molten aluminium alloy i.e. the amount at which the level of metal inside the filling portion is the same as that in the metal bath, the tube 46 has neutral buoyancy, ie it just floats. This prevents the tube being able to sink in the bath in the case of it becoming detached from the body. In addition, without the floatation collar the tube would be able to move downwards too quickly, and so the filling times measured would be too quick to achieve sufficient resolution in results. The buoyancy of the tube must be tailored for the type of aluminium alloy to be measured.

As the level of the molten aluminium alloy in the tube 46 increases, the metallostatic head driving the molten aluminium alloy through the filter 78 decreases and thus the rate of decrease of the load measured by the load cell 96 will decrease Fig. 12c and 12d illustrate the reduction of load measured as the tube 46 is filled. Therefore, to minimise measurement time, the time taken for the filling portion 14 to become 80% full is used to determine aluminium alloy cleanliness. This time is usually between 5 s and 25 s.

The use of stiff materials and structural stiffening elements 26, 28, 40 in the float help to minimise noise in the load output signal, as do the spring between the hinge plate 50 and the reference plate 18, and the rubber strip 90 in the hinge 52, which act as dampers.

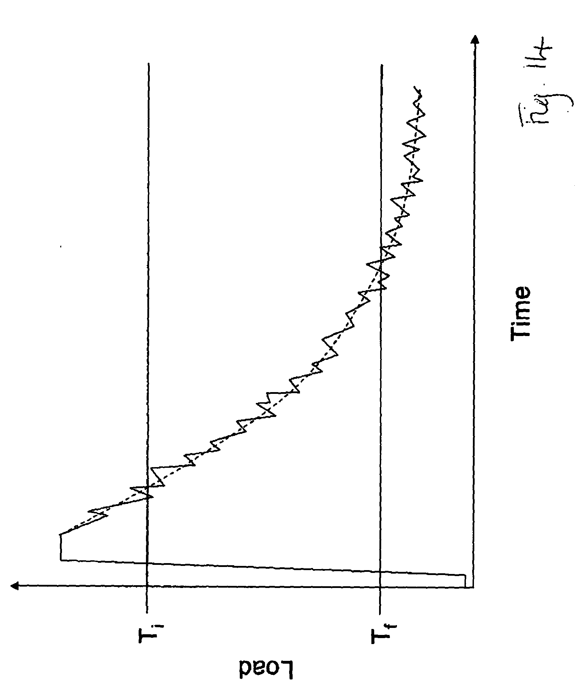

Once the load has decreased past the lower threshold (T_{f}) that indicates the filling portion 14 is 80% full, the software removes the noise from the load-time data to produce a smooth curve (shown as dashed line in Fig. 14), and the filling time is compared to a database of filling times which have been developed for a range of metals and metal qualities. Using this database, the host PC 134 assigns a particular metal quality index to the aluminium alloy being tested. The metal quality index for a valid test is in the range of 1 to 5 (and 6 represents a failed test where the fill threshold was not reached).

Once the index has been determined the result is sent wirelessly to the control box 132 and displayed to the user on the screen. Of the measurements taken, only the filling time and metal quality index assigned are recorded on the host PC 134.

Once the filling portion 14 has been filled to the 80% level, the control box 132 informs the user that the probe 10 can be removed from the molten aluminium alloy. On removing the probe 10, molten aluminium alloy is able to drain back out of the filling portion 14 through the filter 78, thus backflushing the filter 78. As the filter 78 should be substantially horizontal, the backflushing will be substantially uniform across the surface of the filter 78. Backflushing of the filter 78 removes the cake that has built up in the filter 78 due to particle bridging, and thus the filter 78 can be used again.

The force can be checked at any time during the test to ensure it is within a valid range. For example, if the filter is broken, the load-time characteristics would be different to those expected and so the load at a particular time will be outside the valid range, and an error message will be given to the user.

The initial plunge threshold required to start the timer helps the software to recognise tests in which the filter is broken or is not in place - in such a case, filling will start immediately on plunging, thus decreasing the initial upward buoyant force, and preventing the threshold load to initiate timing being reached.

If the time measured for the load to decrease back below the threshold load exceeds a threshold time, the measurement is aborted, as it is deemed the probe 10 is not functioning correctly, for example, the filter 78 may be blocked.

Table 1 compares metal quality index (MQI) results using the probe of this embodiment with density index results, for samples of aluminium alloy which had undergone differing treatment.

**Table 1**

| **Sample No.** | **Treatment** | **Temp / °C** | **Density Index** / % | **MQI** |
|---|---|---|---|---|
| 1 | as melted, settled over night | 730 | 3.86 | 6 |
| 2 | rotary gas treatment 1 min, 300 rpm, 8l/min | 715 | 18.33 | 6 |
| 3 | rotary gas treatment 2 min, 300 rpm, 8l/min | 710 | 14.23 | 5 |
| 4 | rotary gas treatment 2 min, 350 rpm, 8l/min | 715 | 8.48 | 4 |
| 5 | rotary gas treatment 2 min, 350 rpm, 8l/min, 1 hr settling | 730 | 8.46 | 4.5 |
| 6 | rotary gas treatment 2 min, 350 rpm, 8l/min | 712 | 5.73 | 3 |
| 7 | rotary gas treatment 2 min, 350 rpm, 8l/min, 15 min settling | 730 | 5.71 | 3 |
| 8 | addition of 36 kg ingots, 45 min settling | 727 | 6.54 | 4 |
| 9 | rotary gas treatment 2 min, 350 rpm, 8l/min | 719 | 4.98 | 2.5 |
| 10 | rotary gas treatment 2 min, 350 rpm, 8l/min | 717 | 4.45 | 3 |
| 11 | rotary gas treatment 2 min, 350 rpm, 8l/min, 30 min settling | 723 | 3.42 | 2 |
| 12 | rotary gas treatment 3 min, 350 rpm, 8l/min, 250g flux | 715 | 7.57 | 3 |
| 13 | rotary gas treatment 3 min, 350 rpm, 8l/min, 250g flux | 725 | 7.31 | 2 |
| 14 | rotary gas treatment 5 min, 350 rpm, 8l/min, 20 min settling | 723 | 0.87 | 2.5 |

In order to provide an initial molten metal sample, 200 kg of scrap aluminium alloy, typical of the type used for cylinder head production was melted in an electric resistance furnace and allowed to settle overnight.

The appropriate database for the aluminium alloy being tested was chosen for the MQI reference analysis. At this sensitivity setting an MQI of 2 or less would be suitable for cylinder head production. Similarly, it has been found from experience that a density index of <2 would normally be considered acceptable.

MQI and density index measurements (obtained using the reduced pressure test) were then taken after various metal treatments known (based on conventional wisdom and best practice) to continuously improve the metal quality with respect to >50 µm inclusions. (MQI results are based on averages of several measurements). The cleanliness of the metal would be expected to increase progressing down the table. Each MQI measurement took 25 s to obtain, analyse, and record by date, location and metal condition. Pairs of density index samples were collected manually and processed off line in the normal way and the results collated.

Immediately after the first degassing treatment the density index increased whilst the MQI showed no change at the chosen sensitivity setting. After increasing amounts of treatment both the density index and the MQI values fell. During further treatments the density index and MQI values generally correlate. However, after flux treatment the density index increases significantly, showing the sensitivity of the density index to the presence of flux, whilst the MQI continues to fall, indicating that it is not sensitive to the presence of flux. As the MQI measurement is only sensitive to inclusions in the liquid metal, whilst the density index may be affected by different levels of dissolved hydrogen gas or other non particulate materials such as flux, the MQI measurements therefore give a less ambiguous indication of inclusions in the molten metal after standard metal treatment procedures.

Although mild steel was used to form the body 12 of the float 16 and the tube 46 in this embodiment, other materials with sufficient stiffness may be used, for example titanium. If the material used does not deteriorate rapidly when repeatedly dipped in molten aluminium, it may be used without a coating. If a coating is required, alternative coatings to boron nitride may be used, for example, flame or plasma sprayed magnesium zirconate or nickel aluminide.

It should be seen that by fitting a polynomial curve to the load-time curve, more accurate analysis would be possible. Further, use of a polynomial fitting technique could be used to check whether the data is in an acceptable range - i.e. whether the load-time filling characteristics are as would be expected for a valid test.

### Embodiment 2

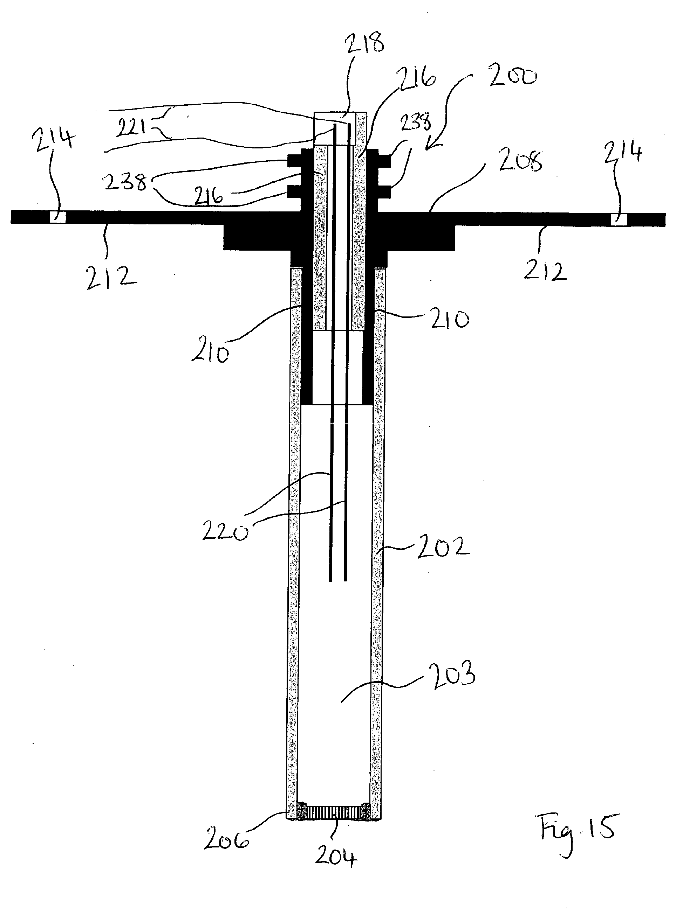

Figures 15 to 17 illustrate this embodiment. The tool or probe 200 comprises a cylindrical filling portion 202, defining a bore 203, with a filter 204 cemented into one end 206 of the filling portion 202. The filling portion 202 is a ceramic fibre tube. A steel tube holder 208 is provided which comprises a cylindrical section 210, and two support arms 212. The arms 212 are diametrically opposed on the circumference of the cylindrical section 210 of the tube holder 208, and both arms 212 extend perpendicularly from the side of the cylindrical section 210. The arms 212 are located about a third of the way down the tube holder 208. Below the arms 212 the cylindrical section 210 is tapered. The tapered section is inserted into the end 208 of the filling portion 202 distal to the filter 204. Each support arm 212 defines an aperture 214 near to the end distal from the filling portion 202.

An inner ceramic fibre tube 216 is provided inside the tube holder 208. A contact block 218 is provided at the top of the inner ceramic fibre tube 216. Two Kanthal wires 220 of equal length are attached to the contact block 218, and the wires 220 depend from the block 218 into the filling portion 202, ending approximately halfway down the filling portion 202. The inner ceramic tube 216 keeps the wires separated from the tube holder 208 The wires are connected to a circuit so that they form a break in the circuit. The contact block 218 is connected to a control box (not shown) via wires 221. The control box is connected wirelessly to a host PC (not shown).

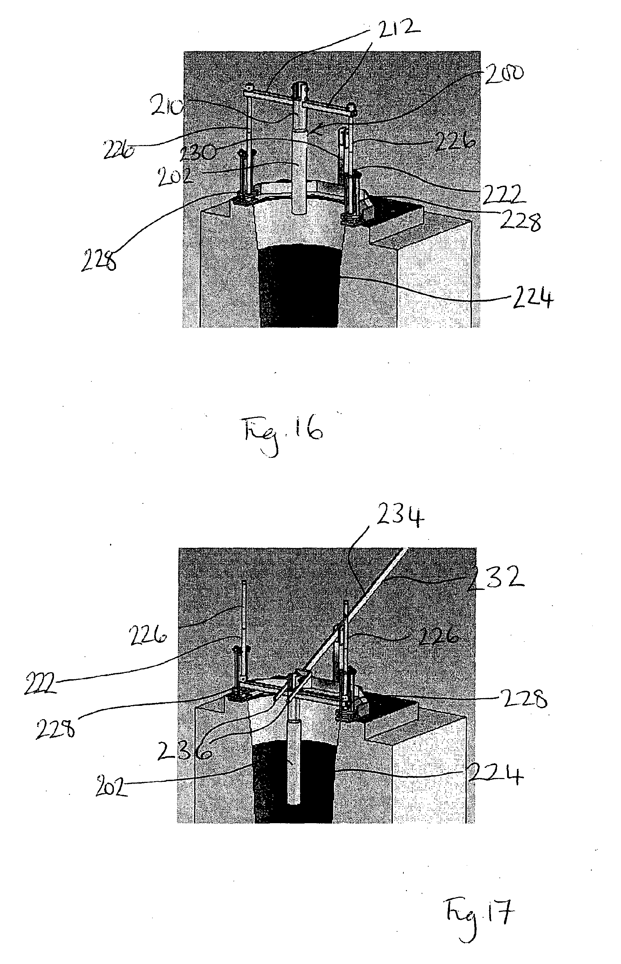

For use, as shown in Fig. 16 and 17, the probe 200 is connected to a rig 222, positioned above a molten iron bath 224. The rig 222 has two positioning arms 226 which extend vertically upwards either side of the iron bath 224. The positioning arms 226 are placed through the apertures 214 in the support arms 212 for the probe 200, so that the probe 200 is able to slide up and down the positioning arms 226. An adjustable stop 228 is provided at the base of each positioning arm 226, so that the probe 200 can be kept in the correct position relative to the level of the molten iron bath 224. The rig 222 includes a lever fulcrum 230 equidistant from the two positioning arms 226.

As for embodiment 1, the probe 200 comprises means to communicate wirelessly with a remote host PC, which has software for calculating iron quality indices.

In use, a lever arm 232 is used to move the probe 200 up and down the positioning arms 226. The lever arm 232 comprises a rod 234, which is forked at one end with two tines 236. The tines 236 of the forked part are straight and parallel to each other. In using the lever arm 232, the tines 236 are positioned either side of the tube holder 208, in between two sets of stops 238 on either side of the tube holder 208. The rod 234 of the lever arm 232 rests on the fulcrum 230 and the operator holds the portion of the rod 234 behind the fulcrum 230. The probe 200 is lowered into the molten iron, and a signal is given to the host PC to start timing. Molten iron enters the bore 203 of the filling portion 202 through the filter 204. The wires 220 act as a measuring device and when the level of the molten iron reaches the wires 220, the molten iron provides a path between the wires 220 to complete the circuit and a signal is communicated to the host PC and the timer is stopped. The software on the host PC can compare the time recorded against a database for various metals and metal qualities, and provide an index of the metal quality, which is communicated to the control box, for display to the operator.

### Embodiment 3

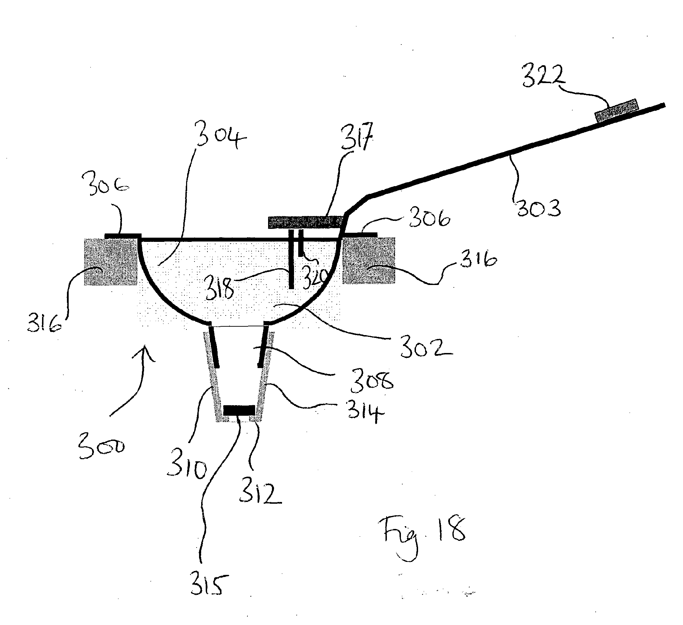

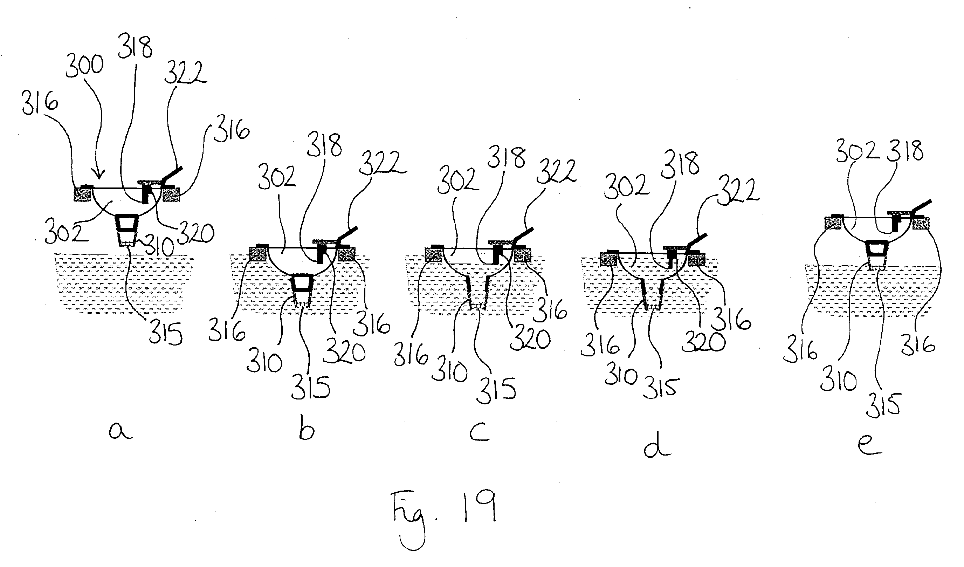

This embodiment is illustrated in Fig. 18 and 19. The probe 300 comprises a filling portion 302 and a placement member 303 attached to the top of the filling portion 302. The filling portion 302 comprises a circular bowl 304, with a lip 306 around the top edge, and a tapered tube 308 depending from the bottom of the bowl 304, with a cup 310 fixed to the tapered tube 308. The cup 310 has a flat base 312 with an orifice therein and a tapered side 314 and is held in place around the tube 308 using molten metal in the same manner as the cup in embodiment 1. There is a filter 315 in the orifice in the base of the cup 310. An annular float 316 is positioned around the bowl 304 of the filling portion 302, so that the lip 306 of the bowl 304 rests on the top of the float 316.

A protruding arm 317 extends from the placement member 303 close to the top of the bowl 304, over the bowl 304. Two wires 318, 320 depend from the arm 316. One wire 318 is longer than the other wire 320. The longer wire 318 extends to approximately two thirds the depth of the bowl 304 and the short wire 320 extends approximately one quarter the depth of the bowl 304. The wires 318, 320 are connected to a control box 322 at the end of the placement member 303 distal to the filling portion 302. The control box 322 is connected wirelessly to a host PC (not shown).

In use, as shown in Fig. 19a to 19b, the probe 300 is placed in molten aluminium and supported by the float 314 which is buoyant in the molten aluminium. The molten aluminium enters the filling portion 302 through the filter 315 and when the level of the molten aluminium reaches the longer wire 318, as shown in Fig 19c, this is detected by the control box 322, and a signal is sent to the host PC, and the host PC starts timing. The filling portion 302 continues to be filled and when the level of the molten aluminium reached the shorter wire 320 the control box 322 detects this and a further signal is sent to the host PC, and the host PC stops timing. The host PC can determine the rate of filling by the time difference between the two signals that are received. The software on the host PC can compare the time recorded against a database for various metals and metal qualities, and provide an index of the metal quality. which is communicated to the control box, for display to the operator.

### Embodiment 4

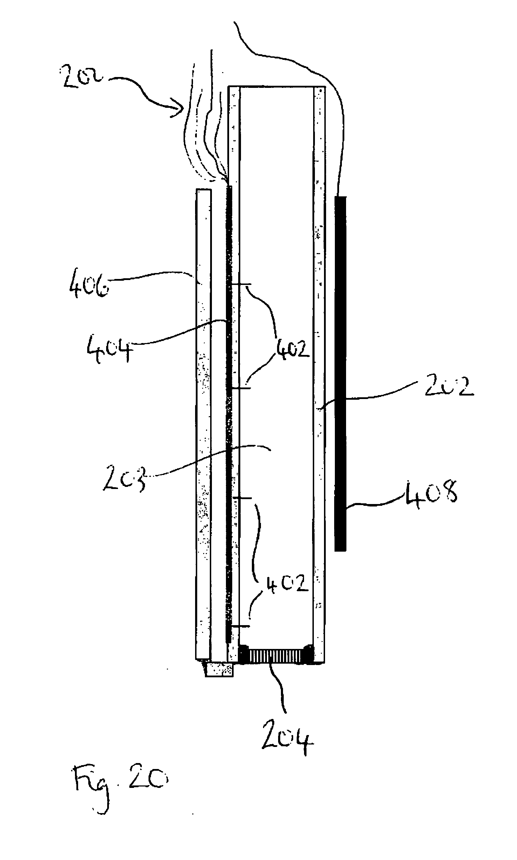

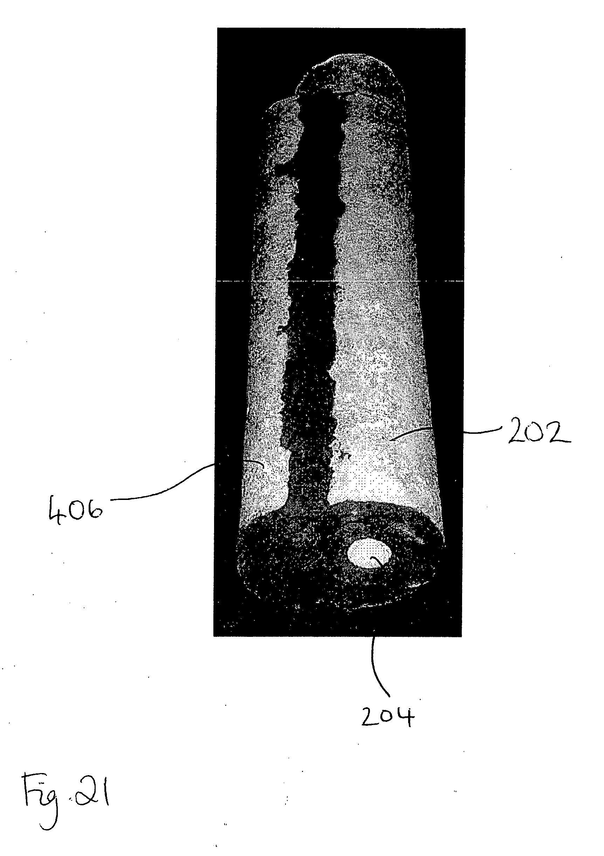

The arrangement of the probe in the fourth embodiment is similar to that of the second embodiment. Only the differences from the second embodiment will be described. The same reference numerals will be used for equivalent features. Fig. 20 and 21 illustrate the probe 200 of this embodiment without the tube holder 208 attached.

The probe 200 does not have two wires 221 as a measuring device as in the second embodiment. Instead, four Kanthal wires 402 are run down the outside of the filling portion 202, each wire 402 penetrates into the bore 203 of the filling portion 202 at a different height to form a contact. The heights at which the wires 402 protrude are equally spaced, the first being just above the bottom of the filling portion 202. The portion of the wires 402 on the outside of the filling portion 202 is covered by a layer of alumina cement 404. The whole arrangement is fired to 500 °C before use. The cement 404 is covered by an extra half section of tube 406 as shown in Fig. 20 and 21, so that the cement 404 covering the contacts does not come in direct contact with the molten aluminium when the probe is submerged, which would result in current leakage through the cement 404. A graphite common rod contact 408 is provided outside the filling portion 202 for submersion in the molten aluminium in the bath. The wires 402 constitute one electrode and the wires 402 and the common rod contact 408 define a break in a circuit.

In use, each time the molten aluminium reaches a wire contact 402, a circuit is completed between the contact 402 and the rod contact 408, and this is detected by the control box and a signal is sent to the host PC. By recording the times between signals, and comparing these times to a database for various metals and metal qualities, the host PC can provide an index of the metal quality, which is communicated to the control box, for display to the operator.

Providing a range of contact points as in this embodiment allows a better chance of detecting any anomalies in flow behaviour than using just two contact points.

### Embodiment 5

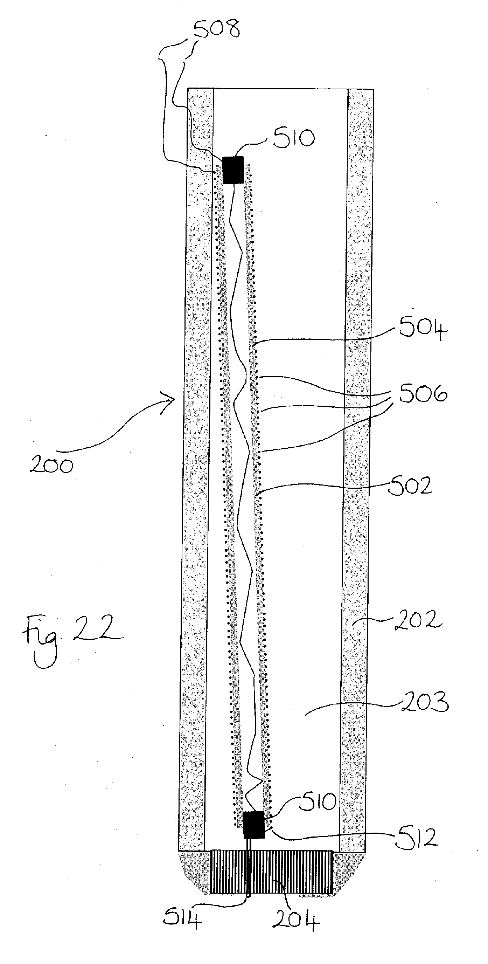

The arrangement of the probe in the fifth embodiment is similar to that of the second embodiment. Only the differences from the second embodiment will be described. The same reference numerals will be used for equivalent features. Fig. 22 illustrates the probe 200 of this embodiment without the tube holder 208 attached.

The probe 200 does not have two wires 221 as a measuring device as in the second embodiment. Instead, a resistance element 502 is used as a measuring device. The resistance element 502 comprises a lintex tube 504 with a length of nichrome wire 506 coiled around it. One end of the wire 506 passes back through the middle of the tube 504 so that the free ends 508 of the wire 506 are both located at the same end of the tube 504. The ends of the tube 504 are blocked with graphite plugs 510 to prevent molten aluminium being able to enter the tube 504. The resistor 502 is placed inside the filling portion 202 with the free ends 508 of the wire 506 at the top, connected to the control box. At the other end 512 of the resistor 502, the wire 506 at the end of the coiled section is passed through the filter 204 and back to form a loop 514 before entering the tube 504.

In use, on submersion of the probe 200 the molten aluminium contacts the loop of wire 514 which passes through the filter 204, and the resistance of the resistance element 502 decreases and acts as a signal for the host PC 134 to start timing. As the molten aluminium rises in the filling portion 202, the resistance measured continues to decrease. Thus a continuous output of fill level against time is achieved. As for embodiment 1, a polynomial is then fitted to the curve and used to calculate the filling time. The filling time is compared to a database of filling times for a range of metals and metal qualities, and a metal quality index is assigned to the sample. As this embodiment provides a continuous output, any anomalies in flow behaviour will not detected as the polynomial will no match any of those in the database.

### Embodiment 6

The arrangement of the probe 200 in the sixth embodiment is similar to the fourth embodiment. Only the differences from the fourth embodiment will be described. The same reference numerals will be used for equivalent features.

The probe 200 of this sixth embodiment is illustrated in Fig. 22. Instead of wire contacts 402 as a measuring device, as in the fourth embodiment, this embodiment has a series of seven equally spaced pin contacts 602, which protrude through the wall of the filling portion 202, into the bore 203, the heads 604 of the pins 602 located on the outside of the filling portion 202. A resistance sensor 606, comprising a coil 608 of resistance wire around a tube 610, is positioned in contact with the pin heads 604. The return wire from the coil runs through the centre of the tube 610. In use, when the molten aluminium makes contact with the two lowest pins 602, the resistance sensor 606 will detect a decrease in resistance. As each further pin is contacted by the molten aluminium, a further decrease in resistance is registered. Signals are sent to the host PC when the resistance decreases. As for embodiment 4, by recording the time between signals, and comparing these times to a database for various metals and metal qualities, the host PC can provide an index of the metal quality, which is communicated to the control box, for display to the operator.

The resistance sensor in this embodiment has the advantage that it does not come into contact with the molten aluminium. It therefore has a longer cycle life than the contacts used in other embodiments, being suitable for being used more than twenty times, compared with three to four times for the fourth embodiment and only once for the fifth embodiment.

### Embodiment 7

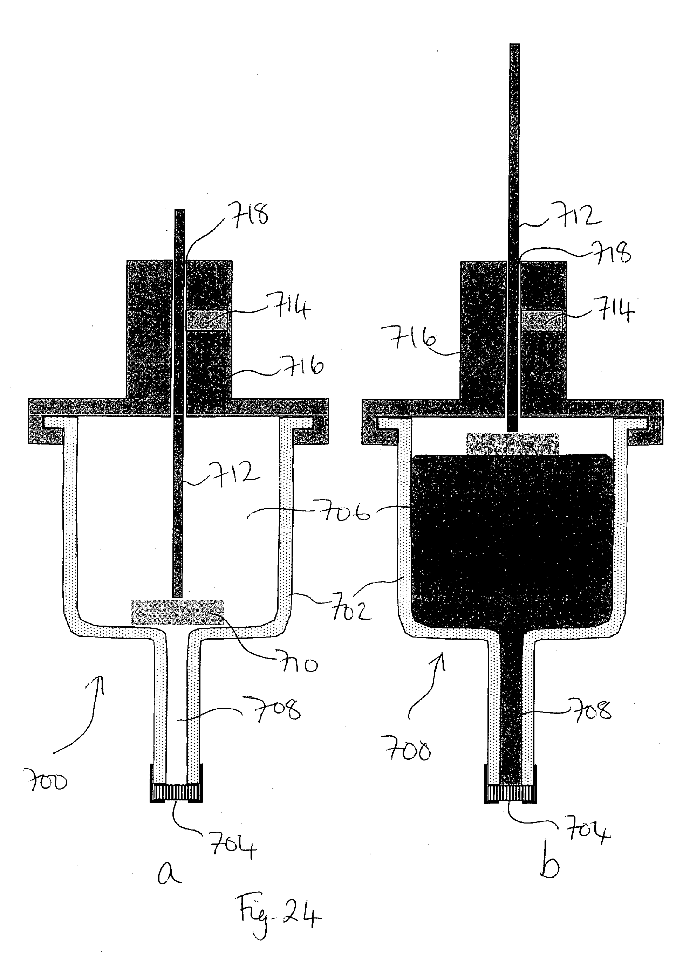

The probe 700 of this embodiment is shown in Fig. 24a and 24b. The probe comprises a filling portion 702 with a filter 704 at the bottom. The filling portion 702 comprises a cylindrical bowl portion 706 and a cylindrical tube portion 708 between the bowl portion 706 and the filter 704. The tube portion 708 has an inside diameter of 20 mm and a length of 60 mm. The bowl portion 706 is uniform in cross section and has an inner diameter of 100 mm.

The means for measuring the rise of molten aluminium in the filling portion 702 is a circular float 710 with a vertically upstanding rod 712 attached, and an optical sensor 714. A lid 716 covers the filling portion 702. The lid 716 defines a long tubular aperture 718, through which the rod 712 attached to the float 710 passes. The lid 716 supports the rod 712, keeping it substantially vertical. The optical sensor 714 is mounted on the lid 716 so that it views the rod 712 in the tubular aperture 718. There rod 712 is smaller in diameter than the tubular aperture 718 so that the inside of the filling portion 702 is at ambient pressure.

In use, as the level of the molten aluminium rises, the float 710 also rises, pushing the rod 712 upwards. The optical sensor 714 is able to measure the displacement of the rod 712. Thus a continuous output of fill level against time is achieved. As for embodiment 1, a polynomial is then fitted to the curve and used to calculate the filling time. The filling time is compared to a database of filling times for a range of metals and metal qualities, and a metal quality index is assigned to the sample. As this embodiment provides a continuous output, any anomalies in flow behaviour will be detected as the polynomial will no match any of those in the database.

The measuring means shown in the above embodiments can be used in systems with floats, as discussed in embodiment 1, or systems with rigs, as discussed in embodiment 2.

Although the probes described in embodiments 2 to 7 are manually operated, it can be seen that robotic operation could also be used.

Location of the probe 200 at the correct position relative to the surface of the molten iron could be carried out automatically, for example by using location means which are electrical contacts located on the outside of the probe, which detect the iron level in relation to the filter position.

The filters used in the above embodiments were ceramic foam filters. However, rigid media, extruded, pressed, or mesh filters could all also be used.

Embodiments 1, and 3 to 7 describe measurement of the cleanliness of molten aluminium, but could be equally well applied to the cleanliness of any molten metal provided materials used withstand heat conditions for the molten metal, and the database of filling times and metal cleanliness indices includes entries for that particular metal. Likewise, although embodiment 2 describes measurement of molten iron, it could also be used to measure other molten metals, if suitable materials were used in the tool.

## Claims

1. A portable measuring tool for determining the quality of molten metal, the tool having a body comprising a filling portion for submersion in the molten metal in use, the filling portion comprising a filter to allow ingress of molten metal into the filling portion after at least partial submersion of the filling portion in molten metal, the tool further comprising a measuring device to measure molten metal characteristics in the filling portion, wherein the body is buoyant in the molten metal and a datum is provided on the tool for use by the measuring device in determining a molten metal characteristic in the filling portion, thereby to enable an indication of metal quality to be determined.

2. A tool as claimed in claim 1, wherein the body comprises floatation means to ensure buoyancy and a consistent plunge depth of the filter in the molten metal.

3. A tool as claimed in claim 1 or claim 2, wherein the filling portion is open to the atmosphere in use so that the pressure inside is the ambient pressure above the molten metal such as atmospheric pressure, thus enabling molten metal to enter the filling portion as a result of the difference between the head of molten metal around the tool and the head of metal in the filling portion, in other words the metallostatic head.

4. A tool for determining the quality of molten metal, the apparatus comprising a body for at least partial submersion in the molten metal, the body comprising a filling portion, a filter being provided to enable ingress of molten metal into the filling portion on at least partial submersion of the body, the filling portion is open so that the pressure inside is the ambient pressure above the molten metal such as atmospheric pressure, thus enabling molten metal to enter the filling portion as a result of the difference between the head of molten metal around the tool and the metal in the filling portion, in other words the metallostatic head, a measuring device being provided to measure the molten metal characteristics in the filling portion, thereby to enable an indication of metal quality to be determined.

5. A tool as claimed in any preceding claim, wherein the measuring device comprises means to measure the amount of molten metal in the filling portion.

6. A tool as claimed in any preceding, wherein the tool enables the time to at least partially fill the filling portion to be determined.

7. A tool as claimed in any preceding claim, wherein a datum is provided on the body for use by the measuring device in determining the amount of metal in the filling portion.

8. A tool as claimed in any preceding claim, wherein the filling portion is able to move relative to the body.

9. A tool as claimed in claim 8, wherein the filling portion is attached to the body via a hinge, and preferably wherein the hinge allows substantially vertical movement of the filling portion relative to the body.

10. A tool as claimed in any preceding claim, wherein the measuring device is adapted to measure displacement of the filling portion relative to the body.

11. A tool as claimed in any preceding claim, wherein the datum is provided as a first reference against subsequent filling by the molten metal, and subsequent references can be used to determine the amount of molten metal in the filling portion in a non-continuous measuring process, and preferably wherein the measuring device comprises a pair of electrical contacts, and more preferably wherein the electrical contacts are located at the same height above the filter, or wherein the electrical contacts are located at different heights above the filter compared with the other contact.

12. A tool as claimed in any preceding claim, wherein a float is provided inside the filling portion, the float being suitable for floatation on top of the molten metal which fills the filling portion, and preferably wherein a rod is attached to the float extending substantially vertically upwards from the float, and more preferably wherein the measuring means may comprise an optical sensor, the optical sensor being able to detect displacement of the rod relative to the sensor.

13. A tool as claimed in any preceding claim, wherein the filling portion can be filled with at least 1 kg of molten metal, more preferably at least 1.5 kg of molten metal, and most preferably at least 2 kg of molten metal, or wherein the filter is masked to define the area of filter through which molten metal can flow, and preferably wherein the unmasked filter area on the outside of the filling portion is equivalent to a circular area with a diameter of between about 10 mm and about 30 mm.

14. A tool as claimed in claim 13, wherein the filter pore size, percentage porosity of the filter, area of the mask and depth of submersion of the probe are such that the filling portion can fill sufficiently for metal quality to be determined in less than 60 s, preferably less than 30 s.

15. A tool as claimed in any preceding claim, wherein the filter is positioned so as to enable egress of liquid from the filling portion, thus backflushing the filter, and preferably wherein the filling portion is adapted such that backflushing of the filter occurs when the tool is removed from the molten metal.
